# EUROPEAN PATENT SPECIFICATION

(11) **EP 2 269 680 B1**
(45) Date of publication and mention of the grant of the patent: **19.09.2012**
(21) Application number: 09008514.3
(22) Date of filing: 30.06.2009
(51) Int. Cl.: A61M 16/10, A61M 16/16

(54) **Respiratory gases humidification system with a uniform humidity level**
System zum Anfeuchten von Atemgasen mit einem konstanten Feuchtigkeitsgrad
Système d'humidification des gaz respiratoires avec un constant niveau d'humidité

(43) Date of publication of application: 05.01.2011
(73) Proprietor: Covidien AG, 8212 Neuhausen am Rheinfall (CH)
(72) Inventor: Zucchi, Giuseppe, 41039 S. Possidonio (MO) (IT); Resca, Daniele, 41038 San Felice sul Panaro (MO) (IT)
(74) Representative: Olsson, Carl H.S.

(56) References cited:
- EP-A1- 2 039 387
- WO-A1-91/19527
- WO-A1-03/099366
- DE-A1- 19 808 590
- US-A- 5 482 031
- US-A- 5 769 071
- US-A- 6 095 505

## Description

The invention relates to a humidifier for humidifying a gas in a respiratory circuit as known from DE 198 08 590 A1 which will be described in further detail below.

In respiratory therapy to patients who are unable to breathe on their own, the physiological function of natural humidification of inspired air performed by upper airways is bypassed, and through the ventilation, the dry air is channeled directly to the bronchi. In this situation the need to moisten the air supplied to the patient in order to simulate as close as possible the natural condition is highlighted.

Currently the active humidification of respiratory gases is performed through equipments described below (Fisher & Paykel or DAR HC 2000 humidifiers).

The first of these devices consists of a hot plate housing a reservoir of water. This reservoir is connected directly to the breathing circuit through a gas input and output port. The water content is heated from the heater underneath in order to saturate of humidity the air above, collected from the respiratory gas flow passing through the reservoir. The enrichment of moisture in respiration gas takes place by lapping the surface of the heated water. The volume of saturated air humidity corresponds to the maximum capacity of the reservoir (i.e. the space free of water). If the volume of inspiratory act is far greater than the one of the reservoir, there will be a much richer humidification at the beginning of the act and a poor air moisture content in the final portion. The second device consists of a heating system featuring a jacket housing the cylindrical water reservoir. The reservoir is made of a double wall, enclosing an hollow space, where the water to be evaporated is collected. The inner wall consists of a vapor permeable membrane that faces directly on the gas flow; the outer wall is made on aluminum. The heating envelopment, in contact with the outer wall made of aluminum, causes evaporation of the film water present in the interspace and then the vapor permeates the inner membrane towards the flow of respiratory gases. The device provides an uneven distribution of moisture during the inspiratory act.

In the above systems it is not possible to define exactly the amount of moisture supplied to each patient ventilation cycle, only an approximate value based on the temperatures involved can be calculated. Furthermore it is necessary to heat water in excess in order to have a quantity of vapor in the breathing circuit sufficient to the patient correct humidification. Because of the amount of water involved, these systems have a certain thermal inertia and therefore a slow response to sudden change in humidification settings and a considerable power demand.

U.S. patent 4,038,980 describes a system for instantaneous evaporation of water for humidification of respiratory gases. The system described consists of an evaporation chamber that is inserted directly on the path of inspiration gases. In this system the water feeding system works non-synchron with the respiratory cycle with the result of having a always different moisture concentration for each respiratory act. DE 198 08 590 A1 discloses a humidifying system with an evaporator which is arranged to receive a defined quantity of fluid, vaporize this quantity of fluid and supply it to a respiratory circuit.

Document WO03/099366 discloses a humidifier for humidifying a gas in a respiratory circuit, with an active humidifying means and a passive humidifying means (HME). The active humidifying means is an evaporator comprising a heating device contacting with a flat side a membrane comprising a hydrophilic membrane and a water impermeable membrane, and a perforated metallic plate. A water inlet is provided between the heating device and the membrane.

In is an object of the present invention to provide a respiratory active humidification system combining the use of an evaporation chamber, for continuous steam production, with a system for collection and release of humidity to maintain a uniform humidity level on patient inhaled gases.

This object is solved by a humidifier according to claim 1. The advantages that the system is able to contribute are described below.

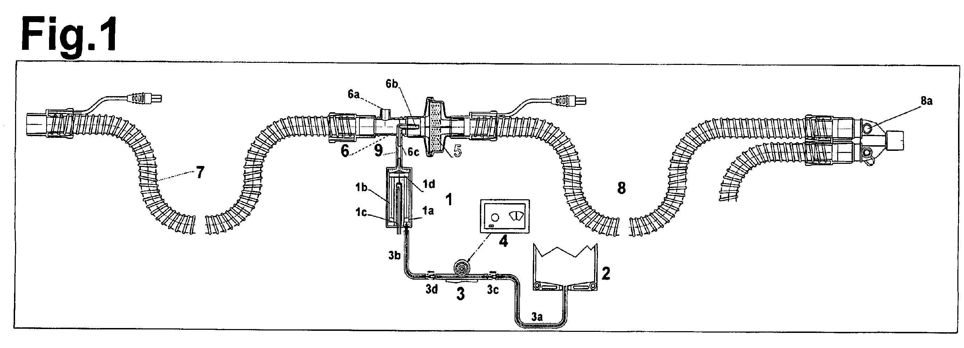

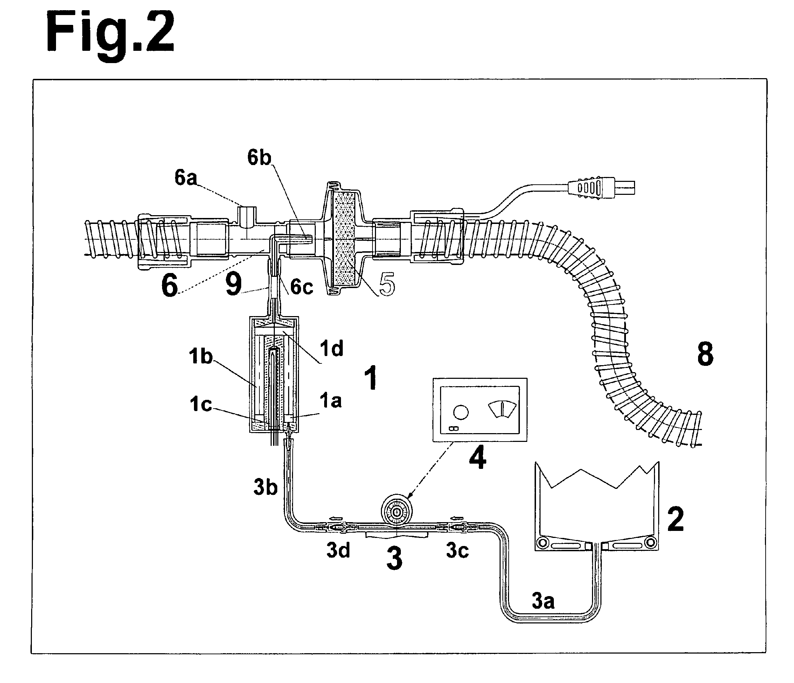

The humidification system of the invention disclosed on fig. 1 and fig. 2, is composed as detailed below:
- An evaporation chamber (1) able to supply water vapour inside the respiratory circuit directly, and determining the constant heating at high temperatures aimed to immediately obtain vapour and, in case the evaporation chamber is not disposable, procure its self-sterilization ;
- A water reservoir (2) connected to the above mentioned evaporation chamber by means of a dosing system (3) allowing the transfer of a defined quantity of water;
- A regulating device (4) allowing the physician to set up the water quantity to be sent to the evaporation chamber with aim to obtain the desired absolute humidity level in the gas inhaled by the patient;
- A humidity exchanger element (5), connected to the inspiratory limb, downstream to the vapour diffusion nozzle (6b), and made of highly hygroscopic material. This element is ideally able to collect all the vapour quantity produced in the expiratory phase (when it is not used), and made of thermal insulating material in order to avoid burns (due to the vapour high temperature) and prevent condensation caused by cooling. The exchanger can be equipped with water display / collection means (not shown). These are useful in case of physician incorrect humidity level set up and an hazardous resistance to flow level could be reached;
- A connector (6) linked to the heated wire inspiratory limb (7) made of material able to resist to the vapour high temperatures and equipped with a temperature probe port (6a). Into the connector a vapour diffusion nozzle (6b)is included, starting perpendicular to the connector and coaxial to the same for its last portion, aimed to deliver vapour coming from the evaporation chamber directly on the humidity exchanger element. The nozzle is connected to the evaporation chamber by means of the connector port (6c) extending outside;
- An heated wire circuit (7), upstream to the evaporation chamber, that pre-heats the ventilation gases and makes them more eager of water (compared to colder gas);
- an heated wire circuit (8), downstream to the humidity exchanger, with aim of maintaining the respiratory gases temperature (avoiding the humidity condensation) and in order to reach the desired temperature ( usually around 37°C), measured by means of a temperature probe placed on port (8a) near to the patient.
- A temperature regulating system, eventually setting different temperatures of the heated wire circuit (not shown).

The system we desire to protect acts as follow:
- The physician sets up, by means of the regulating element (4), the humidification extent he desires to supply to the patient, selecting the water quantity to be delivered;
- This regulations turn into a variation of the water capacity towards the evaporator. The dosing system (3) withdraws water from the reservoir (2) and transfers it to the vaporisation chamber (1a) by means of connecting pipes (3a) and (3b). On the pipes (3a) and (3b) at least two unidirectional valves (3c, 3d)are included, one on the tube connecting the reservoir (2) to the dosing system (3) and another on the tube connecting the latter to the evaporation chamber (1). These check-valves prevent the fluid to come back to the reservoir even when back pressure is present into the evaporation chamber. As shown in fig. 2, a feasible embodiment of the dosing system consists of an eccentric motion mechanism that raising and lowering over the water containing pipe causes its deformation. The deformation results in a change in volume inside the tube. Under crushing, the pressure inside the pipe increases causing the closure of the upstream valve (3c) and simultaneously opening the downstream valve (3d). This results in a transfer of a defined volume of water from the pipe to the evaporation chamber. In the subsequent release of the pipe deflection the pressure inside the tube decreases then causing the closure of the downstream valve (3d) and the opening of upstream valve (3c) withdrawing water from the reservoir.
- the precise amount of metered water flows into the vaporization chamber (1a), distributing on a thin layer over the available area at the base. Then, by means of the high temperature of the chamber it is instantly turned into steam.
- The vapor formed by instantaneous water vaporization flows through the heated channels (1b) distributed evenly around the central heating cartridge (1c). Through this constrained path the steam is further heated to a temperature exceeding 100° C. The superheated steam is finally collected in the upper chamber (1d) from where it is carried outside.
- The steam is then transferred to the respiratory gas flow through a flexible connecting channel (9) made of heat resistant material (such as a silicone tube or connector) linked to the connector (6) through the port (6c). The connector has a small diameter pipe in order to maintain a constant flow of steam, ending at one side into the port (6c) and on the other side in the nozzle which deflects the flow of steam on the humidity exchanger (5) direction. The production of steam obtained by the system object of this invention is stable over time around the level predetermined by the action on the controller (4).

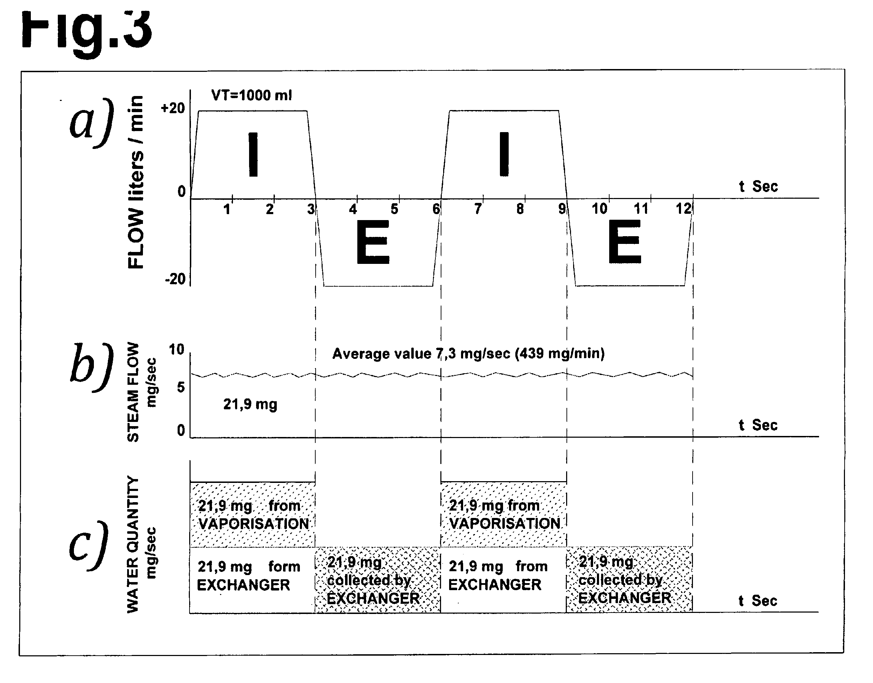

The view of the steam generation trend over time and the combination with the performance of respiratory gas flow is illustrated in Fig. 3 and detailed below:
A - Trend of alternating inspiration / expiration ventilation cycle: each respiratory cycle consists of an inspiratory phase in which a positive volume of air is sent to the patient and an expiratory phase in which the same volume is returned from the patient to the ventilator. The result is a general sinusoidal pattern of the airflow.
B - Trend of the steady flow of steam: the effect of continuous micro-dosing of water to the evaporation chamber results in a nearly constant flow of steam to the inside of the breathing circuit
C - Trend of steam quantity available in the circuit during ventilation: the amount of steam produced during the expiratory phase and gathered by the humidity exchanger element is once again available to the next inspiratory phase. Every inspiratory phase will always contain the same amount of steam.

The humidity exchanger element, consisting of high-efficiency material, serves as a battery of moisture that is able to collect the steam released in the gas flow during the expiratory phase when the gas is not transferred to the patient. Later, during the subsequent inspiratory phase, the preheated gas collects the steam instantaneously produced by the evaporation chamber, (but doesn't get saturated), and flowing to the patient passes through the exchanger, collecting the moisture accumulated during the expiratory phase. The heat exchanger is then dried and made so greedy of moisture for the next phase of steam accumulation.

This also avoids the risk of saturation/occlusion of the exchanger. So the air passing through the inspiratory limb downstream to the exchanger is enriched to the level of humidity set up and maintained, through the thermoregulation of the limb, until the patient is reached.

In the ideal situation the amount of moisture reaching the patient corresponds precisely to the required amount that was set on the regulator. Based on the different initial conditions of ventilation, parameters of a flow of gas and the frequency of inspiration / expiration cycle, the amount of moisture to be supplied to the system can be adjusted accordingly, at the discretion of the physician, plus or minus (inspiration gas saturated or not saturated).

Here are the advantages that the system disclosed is able to contribute:
- The system allows the physician to actually adjust the amount of moisture delivered to the patient through the inspiratory gas;
- due to the small amounts of water to be vaporized the system is extremely fast both when starting evaporation and when it's stopped. The resulting benefit is a rapid response to conditions changes and circumstances of alarm;
- the amount of water that must be provided to the patient through the respiratory gas flow is moved precisely to the evaporation chamber through the use of a water dosing system;
- all metered water is completely vaporized in the evaporation chamber;
- there is no stagnant water in the evaporation chamber and neither in the inspiratory branch, which reduces the risk of bacterial growth;
- in the respiratory circuit the entire amount of water vaporized is completely transferred.
- Then, a fine adjustment of the dosing system allows to precisely determine the amount of vapor that is constantly transferred to the respiratory circuit.
- Since each complete ventilation cycle consists of a phase of inspiration and a phase of expiration opposed one to another, the excess moisture not used during the inspiration phase is collected from the exchanger element placed downstream. In this way the system is able to provide the patient with a gas featuring moisture content almost constant at each ventilation cycle.
- Possibility of self-sterilization of evaporation chamber, if not disposable, through the raising of the temperature.

## Claims

1. A humidifier for humidifying a gas in a respiratory circuit (5, 7, 8), with an evaporator (1) arranged to receive a defined quantity of fluid, vaporize said defined quantity of fluid and supply (1, 6, 9) the vaporized fluid to the respiratory circuit (5, 7, 8), **characterized in that** the evaporator (1) is arranged to provide (6b) the vaporized fluid in the close vicinity of a humidity exchanger (5) arranged in the respiratory circuit (5, 7, 8), and that the evaporator (1) interacts with a vapour diffusion nozzle (6b) connected to an evaporation chamber (1a, 1b, 1 c, 1 d) arranged in the evaporator (1), said nozzle being designed to supply the vaporized fluid directly at the humidity exchanger (5).

2. A humidifier as claimed in claim 1, **characterized in that** a fluid reservoir (2) is connected to the evaporator (1) by means of a dosing system (3), said dosing system (3) being designed to withdraw the defined quantity of fluid from the fluid reservoir (2) and transfer it to the evaporator (1), said defined quantity of fluid is fully vaporized in the evaporator (1).

3. A humidifier as claimed in claim 2, **characterized in that** it comprises a regulator (4) for achieving a defined humidification level of the gas in the respiratory circuit (5, 7, 8), said regulator (4) interacting with the dosing system (3) instructing said dosing system (3) to withdraw the defined quantity of fluid from the fluid reservoir (2) to be vaporized inside the evaporation chamber (1).

4. A humidifier as claimed in claims 2 or 3, **characterized in that** the dosing system (3) comprises a motion movement mechanism deforming a deformable member connected between the fluid reservoir (2) and the evaporator (1), said deformation of the deformable member changing the volume inside the deformable member, wherein a decrease of the volume in the deformable member increase the pressure inside the deformable member closing an upstream valve (3c) and opening of a downstream valve (3d) resulting in a transfer of the defined quantity of fluid to the evaporator (1), and an increase of the volume in the deformable member decrease the pressure inside the deformable member causing the closure of the downstream valve (3d) and the opening of the upstream valve (3c) withdrawing the defined quantity of fluid from the fluid reservoir (2).

5. A humidifier as claimed in claim 4, **characterized in that** the motion movement mechanism is moving eccentrically, where the lowering and raising of the mechanism onto the deformable member causes its deformation.

6. A humidifier as claimed in any of the preceding claims, **characterized in that** said nozzle (6b) is integrated in a connecting tube (9, 6) connected to the evaporator (1), said connecting tube (9, 6) having a tube part (6) extending inside the respiratory circuit, and said nozzle (6b) being arranged at the distal end (6b) of said tube part (6) facing the humidity exchanger (5).

7. A humidifier as claimed in any of the preceding claims, **characterized in that** said evaporator (1) is a single element arranged physically outside the respiratory circuit (5, 7, 8).

8. A humidifier as claimed in claim 4 or 5, **characterized in that** the deformable member is a tube.

9. A humidifier as claimed in any of the preceding claims, **characterized in that** the humidity exchanger (5) is an HME or a filter-HME.

10. A humidifier as claimed in any of the preceding claims, **characterized in that** the fluid is water.

## Patentansprüche

1. Befeuchtungsvorrichtung zum Befeuchten eines Gases in einem Beatmungskreislauf (5, 7, 8), wobei ein Verdampfer (1) zum Aufnehmen einer festgelegten Menge an Fluid, Verdampfen der festgelegten Fluidmenge und Zuführen (1, 6, 9) des verdampften Fluids zum Beatmungskreislauf (5, 7, 8) ausgelegt ist, **dadurch gekennzeichnet, dass** der Verdampfer (1) so ausgelegt ist, dass er für das verdampfte Fluid in enger Nähe zu einem Feuchtigkeitstauscher (5) sorgt (6b), der im Beatmungskreislauf (5, 7, 8) angeordnet ist, und dass der Verdampfer (1) mit einer Dampfdiffusionsdüse (6b) zusammenarbeitet, die mit einer Verdampfungskammer (1a, 1b, 1c, 1d) verbunden ist, welche im Verdampfer (1) angeordnet ist, wobei die Düse so gestaltet ist, dass sie das verdampfte Fluid direkt dem Feuchtigkeitstauscher (5) zuführt.

2. Befeuchtungsvorrichtung nach Anspruch 1, **dadurch gekennzeichnet, dass** ein Fluidvorratsbehälter (2) mit dem Verdampfer (1) durch ein Dosiersystem (3) verbunden ist, wobei das Dosiersystem (3) dafür ausgelegt ist, die festgelegte Fluidmenge aus dem Fluidvorratsbehälter (2) abzuziehen und sie in den Verdampfer (1) zu übertragen, wobei die festgelegte Fluidmenge im Verdampfer (1) vollständig verdampft wird.

3. Befeuchtungsvorrichtung nach Anspruch 2, **dadurch gekennzeichnet, dass** sie einen Regler (4) zum Erreichen eines definierten Feuchtigkeitsgrades des Gases im Beatmungskreislauf (5, 7, 8) umfasst, wobei der Regler (4) mit dem Dosiersystem (3) zusammenwirkt, indem er das Dosiersystem (3) anweist, die festgelegte Fluidmenge aus dem Fluidvorratsbehälter (2) abzuziehen, die in der Verdampfungskammer (1) verdampft werden soll.

4. Befeuchtungsvorrichtung nach Anspruch 2 oder 3, **dadurch gekennzeichnet, dass** das Dosiersystem (3) einen Bewegungsmechanismus umfasst, der ein verformbares Element verformt, welches zwischen dem Fluidvorratsbehälter (2) und dem Verdampfer (1) angeschlossen ist, wobei die Verformung des verformbaren Elementes das Volumen im verformbaren Element ändert, wobei eine Verringerung des Volumens im verformbaren Element den Druck im verformbaren Element erhöht, was ein vorgelagertes Ventil (3c) schließt und ein nachgelagertes Ventil (3d) öffnet, was zu einer Übertragung der festgelegten Fluidmenge in den Verdampfer (1) führt, und wobei eine Erhöhung des Volumens im verformbaren Element den Druck im verformbaren Element verringert, wodurch das nachgelagerte Ventil (3d) geschlossen wird und das vorgelagerte Ventil (3c) geöffnet wird, wobei die festgelegte Fluidmenge aus dem Fluidvorratsbehälter (2) abgezogen wird.

5. Befeuchtungsvorrichtung nach Anspruch 4, **dadurch gekennzeichnet, dass** der Bewegungsmechanismus sich exzentrisch bewegt, wobei das Senken und Anheben des Mechanismus auf das verformbare Element dessen Verformung verursacht.

6. Befeuchtungsvorrichtung nach einem der vorherigen Ansprüche, **dadurch gekennzeichnet, dass** die Düse (6b) in ein Verbindungsrohr (9, 6) integriert ist, welches mit dem Verdampfer (1) verbunden ist, wobei das Verbindungsrohr (9, 6) einen Rohrteil (6) hat, der sich innerhalb des Beatmungskreislaufs erstreckt, und wobei die Düse (6b) am distalen Ende (6b) des Rohrteils (6) angeordnet ist, der dem Feuchtigkeitstauscher (5) gegenüberliegt.

7. Befeuchtungsvorrichtung nach einem der vorherigen Ansprüche, **dadurch gekennzeichnet, dass** der Verdampfer (1) ein Einzelelement ist, das physisch außerhalb des Beatmungskreislaufs (5, 7, 8) angeordnet ist.

8. Befeuchtungsvorrichtung nach Anspruch 4 oder 5, **dadurch gekennzeichnet, dass** das verformbare Element ein Rohr ist.

9. Befeuchtungsvorrichtung nach einem der vorherigen Ansprüche, **dadurch gekennzeichnet, dass** der Feuchtigkeitstauscher (5) ein Wärme- und Feuchtigkeitstauscher (HME) oder ein Filter-HME ist.

10. Befeuchtungsvorrichtung nach einem der vorherigen Ansprüche, **dadurch gekennzeichnet, dass** das Fluid Wasser ist.

## Revendications

1. Humidificateur pour humidifier un gaz dans un circuit respiratoire (5, 7, 8), comprenant un évaporateur (1) qui est agencé de manière à recevoir une quantité de fluide définie, à vaporiser ladite quantité de fluide définie, et à fournir (1, 6, 9) le fluide vaporisé au circuit respiratoire (5, 7, 8), **caractérisé en ce que** l'évaporateur (1) est agencé de manière à fournir (6b) le fluide vaporisé dans le voisinage proche d'un échangeur d'humidité (5) qui est disposé dans le circuit respiratoire (5, 7, 8), et **en ce que** l'évaporateur (1) interagit avec une buse de diffusion de vapeur (6b) connectée à une chambre d'évaporation (1a, 1b, 1e, 1d) qui est disposée dans l'évaporateur (1), ladite buse étant conçue de manière à fournir le fluide vaporisé directement à l'échangeur d'humidité (5).

2. Humidificateur selon la revendication 1, **caractérisé en ce qu'**un réservoir de fluide (2) est connecté à l'évaporateur (1) au moyen d'un système de dosage (3), ledit système de dosage (3) étant conçu de manière à aspirer la quantité de fluide définie hors du réservoir de fluide (2) et à la transférer à l'évaporateur (1), ladite quantité de fluide définie étant complètement vaporisée dans l'évaporateur (1).

3. Humidificateur selon la revendication 2, **caractérisé en ce qu'**il comprend un régulateur (4) pour atteindre un niveau d'humidification défini du gaz dans le circuit respiratoire (5, 7, 8), ledit régulateur (4) interagissant avec le système de dosage (3) pour donner l'instruction audit système de dosage (3) d'aspirer hors du réservoir (2) la quantité de fluide définie à vaporiser à l'intérieur de la chambre d'évaporation (1).

4. Humidificateur selon la revendication 2 ou 3, **caractérisé en ce que** le système de dosage (3) comprend un mécanisme de déplacement en mouvement pour déformer un élément déformable qui est connecté entre le réservoir de fluide (2) et l'évaporateur (1), ladite déformation de l'élément déformable modifiant le volume à l'intérieur de l'élément déformable, dans lequel une diminution du volume de l'élément déformable augmente la pression à l'intérieur de l'élément déformable pour fermer une soupape située en amont (3c) et ouvrir une soupape située en aval (3d), entraînant un transfert de la quantité de fluide définie dans l'évaporateur (1), et une augmentation du volume de l'élément déformable diminue la pression à l'intérieur de l'élément déformable, entraînant la fermeture de la soupape située en aval (3d) et l'ouverture de la soupape située en amont (3c), aspirant de ce fait la quantité de fluide définie hors du réservoir de fluide (2).

5. Humidificateur selon la revendication 4, **caractérisé en ce que** le mécanisme de déplacement en mouvement se déplace de façon excentrique, dans lequel l'abaissement et l'élévation du mécanisme sur l'élément déformable entraînent sa déformation.

6. Humidificateur selon l'une quelconque des revendications précédentes, **caractérisé en ce que** ladite buse (6b) est intégrée dans un tube de connexion (9, 6) qui est connecté à l'évaporateur (1), ledit tube de connexion (9, 6) comprenant une partie de tube (6) qui s'étend à l'intérieur du circuit respiratoire, et ladite buse (6b) étant agencée à l'extrémité distale (6b) de ladite partie de tube (6) qui fait face à l'échangeur d'humidité (5).

7. Humidificateur selon l'une quelconque des revendications précédentes, **caractérisé en ce que** ledit évaporateur (1) est un élément unique qui est agencé physiquement à l'extérieur du circuit respiratoire (5, 7, 8).

8. Humidificateur selon la revendication 4 ou 5, **caractérisé en ce que** l'élément déformable est un tube.

9. Humidificateur selon l'une quelconque des revendications précédentes, **caractérisé en ce que** l'échangeur d'humidité (5) est un échangeur de chaleur et d'humidité (HME), ou un HME à filtre.

10. Humidificateur selon l'une quelconque des revendications précédentes, **caractérisé en ce que** le fluide est l'eau.
